# EUROPEAN PATENT SPECIFICATION

(11) **EP 2 213 317 B1**
(45) Date of publication and mention of the grant of the patent: **02.04.2014**
(21) Application number: 10152449.4
(22) Date of filing: 02.02.2010
(51) Int. Cl.: A61M 1/34

(54) **Ozone-therapy blood treatment**
Ozontherapie-Blutbehandlung
Traitement sanguin de thérapie à l'ozone

(30) Priority: 03.02.2009 IT BO20090049
(43) Date of publication of application: 04.08.2010
(73) Proprietor: Bellco S.r.l., Mirandola (IT)
(72) Inventor: Bergamini, Guido, 41037, MIRANDOLA (IT)
(74) Representative: Bergadano, Mirko

(56) References cited:
- EP-A2- 0 451 429
- DE-A1- 3 307 830
- US-A- 2 887 107
- US-A- 6 027 688
- US-A1- 2003 073 945

## Description

The present invention relates to an ozone-therapy blood treatment.

The therapeutic effects of ozone have been known for many years now; when ozone is brought into contact with the blood or with other tissues of the organism, it does not present toxicity only at extremely low dosage levels.

In particular, ozone therapy has been applied for a long time in antalgic therapies and in the reduction of inflammation at the level of articulations and vertebral disks via insufflation of a mixture of oxygen and ozone gases.

It has moreover been highlighted how the treatment of blood with ozone yields particularly interesting pharmacological responses. For example, autohaemotherapy with ozone has been proposed in various human diseases on account of its multiple therapeutic actions, amongst which the best known are: activation of the immune system in infective illnesses; release of growth factors; activation of the immune system and killer action on cancer cells.

Recently, ozone therapy has been practised by the use of exchangers in the EBOO (Extracorporeal Blood Oxygenation and Ozonation) technique. EBOO is a new therapeutic technique, which is based upon the contact of the blood with certain gases, and is performed with a method altogether similar to that of haemodialysis, by causing a certain amount of gases to flow into the aqueous compartment instead of the dialysing solution and using particular filters useful for the passage of gas.

Such a technique, even though representing a progress in ozone blood therapy, entails, however, a series of problems regarding the high costs and the complications due to the choice of the vascular access suitable for obtaining the blood flow and to coagulation, of the devices themselves.

Document D1= US 2003/073945 A1 discloses a machine comprising an extracorporeal circuit comprising a filter, an arterial line, designed for transport of the blood from the patient to the filter, a venous line, designed for transport of the blood from the filter to the patient, an ozone generator and a replenishing solute circuit, designed for transport isotonic solution with aqueous ozone from the ozone generator to the venous line.

The aim of the present invention is to provide a machine for ozone-therapy blood treatment, the technical characteristics of which are such as to overcome in a simple and inexpensive way the problems of the known art.

The subject of the present invention is a machine for ozone-therapy blood treatment, the essential characteristics of which are given in Claim 1, and the preferred and/or auxiliary characteristics of which are given in Claims 2-9.

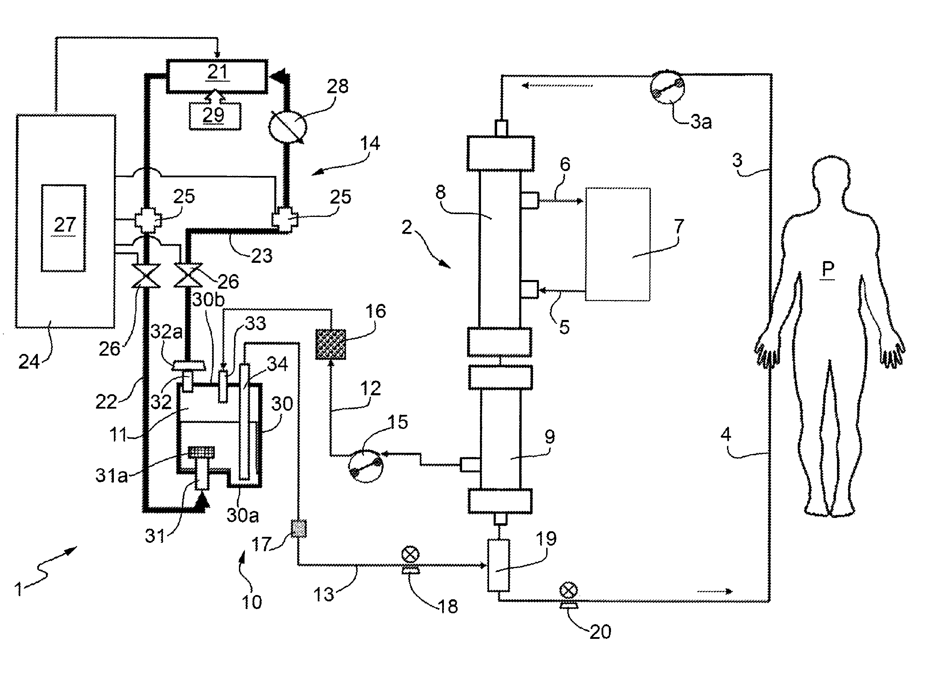

For a better understanding of the invention an embodiment thereof is described in what follows purely by way of illustrative and non-limiting example with the aid of the figure of the annexed drawings, which is a schematic view of the extracorporeal blood circuit for the ozone-therapy blood treatment forming the subject of the present invention.

Designated as a whole by 1 in the figure is the extracorporeal blood circuit of a patient P. The circuit 1 comprises a dialysis filter 2, an arterial line 3, which is responsible for transport of the blood from the patient P to the filter 2, a pump 3a, applied to the arterial line 3 for guaranteeing movement of the blood, a venous line 4, which is responsible for transport of the blood from the filter 2 to the patient P, an inflow branch 5 and an outflow branch 6 for inflow and outflow of a dialysing solution respectively into and from the filter 2, and a device 7 for preparing the dialysing solution connected at least to the inflow branch 5.

The filter 2 consists of a pair of filters 8 and 9 set in series, each of which is made up of capillary tubes within which the blood flows. Taking as reference the direction of the flow of the blood of the patient P, the first filter 8 is responsible for haemodialysis by means of diffusive and convective phenomena with the dialysing solution, whilst the second filter 9 is responsible for extraction by ultrafiltration of a plasma component. By "plasma component" is meant either plasmatic water or whole plasma according to the permeability of the capillary tubes used.

The circuit 1 comprises an ozone-therapy circuit 10, in which the plasma component taken from the filter 9 is made to react with the ozone and subsequently re-infused into the patient P.

The circuit 10 comprises a reaction chamber 11, in which the ozone is made to react with the plasma component, a delivery line 12, responsible for transport of the plasma component from the second filter 9 to the reaction chamber 11, a return line 13, responsible for transport of the plasma component that has reacted with the ozone from the reaction chamber 11 to the venous line 4, and an assembly for preparation and movement of the ozone 14.

The delivery line 12 comprises a pump 15 and a sorbent cartridge 16 useful for purification of the plasma component from toxins.

The return line 13 comprises a microbubble trap 17 and a microbubble alarm 18, and is connected to the venous line 4 by means of a venous drip 19 useful for mixing the plasma component with the blood. In particular, for obvious reasons of safety, a further microbubble alarm 20 is set along the venous line 4 downstream of the venous drip 19.

The assembly for preparation and movement of the ozone 14 comprises a device 21 for preparing the ozone, an inlet line 22 and an outlet line 23 for inflow and outflow of the ozone, respectively, into and from the reaction chamber 11.

Furthermore, the assembly for preparation and movement of the ozone 14 comprises a command and control unit 24, connected to the preparing device 21, two concentration sensors 25, set respectively on the inlet line 22 and on the outlet line 23 and designed to transfer the data detected to the command and control unit 24, and two electromechanical valves 26, set respectively on the inlet line 22 and on the outlet line 23 and governed by the command and control unit 24 itself.

In use, the concentration sensor 25 set on the inlet line 22 reads the concentration of ozone introduced into the reaction chamber 11, whilst the concentration sensor 25 set on the outlet line 23 reads the residual concentration of ozone, and the difference of the two values yields the amount of ozone that has reacted with the plasma component.

The concentration of ozone may be varied manually or in a completely automatic way using a software that stores the dose as a function of the principal parameters of the patient P storing the data in a patient sheet 27. Furthermore, since the concentration sensors 25 are also responsible for reading the inflow and outflow pressure, the command and control unit 24 acts on the electromechanical valves 26 to guarantee control of the pressure within the reaction chamber 11.

The assembly for preparation and movement of the ozone 14 comprises an ozone destroyer 28, set on the outlet line 23 downstream of the concentration sensor 25, and an electronic nose 29, designed to detect any possible exit of ozone from the preparing device 21.

The reaction chamber 11 is defined by a plurality of walls 30 made of ozone-resistant material, such as, for example, polycarbonate, polyurethane, Teflon, and rigid PVC, and comprises a distributing element 31, emerging from a bottom wall 30a and connected to the inlet line 22, an element for outflow of the gases 32, emerging from a top wall 30b and connected to the outlet line 23, an element for inflow of the plasma component 33, emerging from the top wall 30b and connected to the delivery line 12, and a suction element 34, which also emerges from the top wall 30b and is connected to the return line 13.

In particular, the distributing element 31 may be filtering through a microperforated surface 31a, whilst the element for outflow of the gases 32 comprises a water-repellent filter 32a.

According to the ozone-therapy blood treatment performed with the machine of the present invention, the solutes present in the plasmatic water or in the whole plasma react with the ozone and are re-infused into the patient P during dialysis. The solutes thus reacted are able to activate the same physiological reactions that would be activated by a direct reaction of the blood with the ozone.

The treatment performed with the machine of the present invention presents the advantages of excluding the possibility of phenomena of coagulation, of enabling easy modulation of the exchange flow, as well as affording extreme ease of management of the treatment as a whole.

Furthermore, by applying to the patients undergoing dialysis the treatment performed with the machine of the present invention, the duration of the treatment itself is set equal to the duration of the treatment of dialysis, and hence the flows and concentrations may be adequately modulated, thus also facilitating exchange for the entire duration of the treatment.

Finally, the data of the treatment can be stored and, through an appropriate control electronics, will enable calibration of the system for generation and infusion of the gas in relation to the plasma flow generated by the pump 15.

As may prove obvious to a person skilled in the branch, the treatment performed with the machine of the present invention can be implemented irrespective of the presence or otherwise of the dialysis treatment, as instead is described above.

## Claims

1. A machine for ozone-therapy blood treatment, comprising an extracorporeal circuit (1) comprising a filter (9), designed to produce a plasma component from the blood of a patient (P), an arterial line (3), designed for transport of the blood from the patient (P) to the filter (9), a venous line (4), designed for transport of the blood from the filter (9) to the patient (P), a reaction chamber (11), in which the plasma component is made to react with a gas containing ozone, a delivery line (12), designed for transport of the plasma component from the filter (9) to the reaction chamber (11), a return line (13), designed for transport of the plasma component that has reacted with the ozone from the reaction chamber (11) to the venous line (4), and an assembly for preparation and movement of the ozone (14), designed for the production of ozone and for its inlet into the reaction chamber (11).

2. The machine for ozone-therapy blood treatment according to Claim 1, **characterized in that** the delivery line (12) comprises a pump (15) and a sorbent cartridge (16) useful for purification of the plasma component from toxins.

3. The machine for ozone-therapy blood treatment according to Claim 1 or Claim 2, **characterized in that** the return line (13) comprises a microbubble trap (17) and a microbubble alarm (18) and **in that** it comprises a further microbubble alarm (20) set along the venous line (4).

4. The machine for ozone-therapy blood treatment according to any one of the preceding claims, **characterized in that** it comprises a venous drip (19), designed to connect the return line (13) to the venous line (4).

5. The machine for ozone-therapy blood treatment according to any one of the preceding claims, **characterized in that** said assembly for preparation and movement of the ozone (14) comprises a device (21) for preparing the ozone, an inlet line (22) and an outlet line (23) for the inflow and outflow of the ozone, respectively, into and from the reaction chamber (11).

6. The machine for ozone-therapy blood treatment according to Claim 5, **characterized in that** the assembly for preparation and movement of the ozone (14) comprises a command and control unit (24), connected to the preparing device (21), two concentration sensors (25), set respectively on the inlet line (22) and on the outlet line (23) and designed to transfer the data detected to the command and control unit (24), and two electromechanical valves (26), set respectively on the inlet line (22) and on the outlet line (23) and governed by the command and control unit (24) itself.

7. The machine for ozone-therapy blood treatment according to Claim 5 or Claim 6, **characterized in that** the reaction chamber (11) is defined by a plurality of walls (30) made of ozone-resistant material and comprises a distributing element (31), emerging from a bottom wall (30a) and connected to the inlet line (22), an element for outflow of the gases (32), emerging from a top wall (30b) and connected to the outlet line (23), an element for inflow of the plasma component (33), emerging from the top wall (30b) and connected to the delivery line (12), and a suction element (34), which also emerges from the top wall (30b) and is connected to the return line (13).

8. The machine for ozone-therapy blood treatment according to Claim 7, **characterized in that** the distributing element (31) comprises a microperforated surface (31a), and the element for outflow of the gases (32) comprises a water-repellent filter (32a).

9. The machine for ozone-therapy blood treatment according to any one of the preceding claims, **characterized in that** said filter is a dialysis filter (2) comprising a pair of filters (8, 9) set in series with respect to one another; one said first filter (8) being responsible for haemodialysis and one said second filter (9) being responsible for extraction by ultrafiltration of the plasma component.

## Patentansprüche

1. Vorrichtung zur Blutbehandlung im Rahmen einer Ozontherapie, mit einem extrakorporalen Kreislauf (1) mit einem Filter (9) zur Herstellung einer Plasmakomponente aus dem Blut eines Patienten (P), einer arteriellen Leitung (3), zum Transport des Blutes eines Patienten (P) zu dem Filter (9), einer venösen Leitung (4) zum Transportieren des Blutes von dem Filter (9) zum Patienten (P), einer Reaktionskammer (11), in welcher die Plasmakomponente mit einem ozonhaltigen Gas zur Reaktion gebracht wird, einer Zuführleitung (12) zum Transportieren der Plasmakomponente von dem Filter (9) zu der Reaktionskammer (11), einer Rückführleitung (13) zum Transportieren der Plasmakomponente, die mit dem Ozon reagiert hat, von der Reaktionskammer (11) zu der venösen Leitung (4) und einer Baueinheit für die Herstellung und Bewegung des Ozons (14) zur Herstellung des Ozons und dessen Einspeisung in die Reaktionskammer (11).

2. Vorrichtung zur Blutbehandlung im Rahmen einer Ozontherapie nach Anspruch 1, **dadurch gekennzeichnet, dass** die Zuführleitung (12) eine Pumpe (15) und eine für die Reinigung des Blutes von Giftstoffen nutzbare Sorbenskartusche (16) aufweist.

3. Vorrichtung zur Blutbehandlung im Rahmen einer Ozontherapie nach Anspruch 1 oder 2, **dadurch gekennzeichnet, dass** die Rückführleitung eine Mikroblasenfalle (17) und einen Mikroblasenalarm (18) aufweist und dass die Vorrichtung einen weiteren Mikroblasenalarm (20) angeordnet an der venösen Leitung (4) aufweist.

4. Vorrichtung zur Blutbehandlung im Rahmen einer Ozontherapie nach einem der vorhergehenden Ansprüche, **dadurch gekennzeichnet, dass** sie einen venösen Tropf (19) zur Verbindung der Rückführleitung (19) mit der venösen Leitung (4) aufweist.

5. Vorrichtung zur Blutbehandlung im Rahmen einer Ozontherapie nach einem der vorhergehenden Ansprüche, **dadurch gekennzeichnet, dass** die Baueinheit für die Herstellung und Bewegung des Ozons (14) eine Einrichtung (21) zur Herstellung von Ozon, eine Einlassleitung (22) und eine Auslassleitung (23) jeweils zum Einstrom und Ausstrom des Ozons in und aus der Reaktionskammer (11), umfasst.

6. Vorrichtung zur Blutbehandlung im Rahmen einer Ozontherapie nach Anspruch 5, **dadurch gekennzeichnet, dass** die Baueinheit für Herstellung und Bewegung des Ozons (14) eine mit der Herstellungseinrichtung (21) verbundene Befehls- und Regeleinheit (24), zwei jeweils an der Einlassleitung (22) und Auslassleitung (23) angebrachte und für den Transfer von Daten an die Befehls- und Regeleinheit (24) angepasste Konzentrationssensoren (25) und zwei jeweils an der Einlassleitung (22) und Auslassleitung (23) angebrachte und durch die Befehls- und Regeleinheit (24) selbst geregelte elektromechanische Ventile (26), umfasst.

7. Vorrichtung zur Blutbehandlung im Rahmen einer Ozontherapie nach Anspruch 5 oder 6, **dadurch gekennzeichnet, dass** die Reaktionskammer (11) durch eine Mehrzahl an Wänden aus ozonresistentem Material begrenzt ist und ein von einer unteren Wand hervortretendes und mit der Einlassleitung (22) verbundenes Verteilerelement (31), ein von der oberen Wand hervortretendes und mit der Auslassleitung (23) verbundenes Element für den Ausstrom des Gases (32), ein von der oberen Wand hervortretendes und mit der Zuführleitung (12) verbundenes Element für den Einfluss der Plasmakomponente (33) und ein weiteres von der oberen Wand hervortretendes und mit der Rückführleitung (13) verbundenes Saugelement (34) umfasst.

8. Vorrichtung zur Blutbehandlung im Rahmen einer Ozontherapie nach Anspruch 7, **dadurch gekennzeichnet, dass** das Verteilerelement (31) eine mikroperforierte Oberfläche (31 a) umfasst und dass das Element für den Ausstrom des Gases (32) einen wasserabweisenden Filter (32a) umfasst.

9. Vorrichtung zur Blutbehandlung im Rahmen einer Ozontherapie nach einem der vorhergehenden Ansprüche, **dadurch gekennzeichnet, dass** der Filter ein Dialysefilter mit einem Paar zueinander in Serie angeordneten Filtern ist, wobei der erste Filter (8) für eine Hämodialyse verantwortlich und der zweite Filter (9) für eine Extraktion der Plasmakomponente durch Ultrafiltration verantwortlich ist.

## Revendications

1. Machine pour le traitement du sang par ozonothérapie, comprenant un circuit extracorporel (1) comprenant un filtre (9), conçu pour produire un composant plasmatique à partir du sang d'un patient (P), une ligne artérielle (3), conçue pour le transport du sang du patient (P) au filtre (9), une ligne veineuse (4), conçue pour le transport du sang du filtre (9) au patient (P), une chambre de réaction (11), dans laquelle le composant plasmatique est amené à réagir avec un gaz contenant de l'ozone, une ligne de distribution (12), conçue pour le transport du composant plasmatique du filtre (9) à la chambre de réaction (11), une ligne de retour (13), conçue pour le transport du composant plasmatique qui a réagi avec l'ozone de la chambre de réaction (11) à la ligne veineuse (4), et un ensemble pour la préparation et le déplacement de l'ozone (14), conçu pour la production d'ozone et pour son entrée dans la chambre de réaction (11) .

2. Machine pour le traitement du sang par ozonothérapie selon la revendication 1, **caractérisée en ce que** la ligne de distribution (12) comprend une pompe (15) et une cartouche de sorbant (16) utile pour la purification des toxines du composant plasmatique.

3. Machine pour le traitement du sang par ozonothérapie selon la revendication 1 ou la revendication 2, **caractérisée en ce que** la ligne de retour (13) comprend un piège à microbulles (17) et une alarme de microbulles (18) et **en ce qu'**elle comprend une alarme de microbulles supplémentaire (20) placée le long de la ligne veineuse (4).

4. Machine pour le traitement du sang par ozonothérapie selon l'une quelconque des revendications précédentes, **caractérisée en ce qu'**elle comprend un goutte à goutte veineux (19), conçu pour relier la ligne de retour (13) à la ligne veineuse (4).

5. Machine pour le traitement du sang par ozonothérapie selon l'une quelconque des revendications précédentes, **caractérisée en ce que** ledit ensemble pour la préparation et le déplacement de l'ozone (14) comprend un dispositif (21) pour préparer l'ozone, une ligne d'entrée (22) et une ligne de sortie (23) pour l'écoulement d'entrée et l'écoulement de sortie de l'ozone, respectvement, dans et depuis la chambre de réaction (11).

6. Machine pour le traitement du sang par ozonothérapie selon la revendication 5, **caractérisée en ce que** l'ensemble pour la préparation et le déglacement de l'ozone (14) comprend une unité de commande et de contrôle (24), reliée au dispositif de préparation (21), deux capteurs de concentration (25), placés respectivement sur la ligne d'entrée (22) et sur la ligne de sortie (23) et conçus pour transférer les données détectées par l'unité de commande et de contrôle (24), et deux soupapes électromécaniques (26), placées respectivement sur la ligne d'entrée (22) et sur la ligne de sortie (23) et régulées par l'unité de commande et de contrôle (24) elle-même.

7. Machine pour le traitement du sang par ozo-nothérapie selon la revendication 5 ou la revendication 6, **caractérisée en ce que** la chambre de réaction (11) est définie par une pluralité de parois (30) constituées d'un matériau résistant à l'ozone et comprend un élément de distribution (31), émergeant d'une paroi de fond (30a) et relié à la ligne d'entrée (22), un élément pour l'écoulement de sortie des gaz (32), émergeant d'une paroi supérieure (30b) et relié à la ligne de sortie (23), un élément pour l'écoulement d'entrée du composant plasmatique (33), émergeant de la paroi supérieure (30b) et relié à la ligne de distribution (12), et un élément d'aspiration (34), qui émerge également de la paroi supérieure (30b) et est relié à la ligne de retour (13).

8. Machine pour le traitement du sang par ozonothérapie selon la revendication 7, **caractérisée en ce que** l'élément de distribution (31) comprend une surface microperforée (31a), et l'élément pour l'écoulement de sortie des gaz (32) comprend un filtre hydrofuge (32a).

9. Machine pour le traitement du sang par ozo-nothérapie selon l'une quelconque des revendications précédentes, **caractérisée en ce que** ledit filtre est un filtre de dialyse (2) comprenant une paire de filtres (8, 9) placés en série l'un par rapport à l'autre ; un dit premier filtre (8) étant responsible de l'hémodialyse et un dit second filtre (9) étant responsable de l'extraction par ultrafiltration du composant plasmatique.
